# EUROPÄISCHE PATENTSCHRIFT

(11) **EP 2 158 185 B1**
(45) Veröffentlichungstag und Bekanntmachung des Hinweises auf die Patenterteilung: **17.08.2011**
(21) Anmeldenummer: 08761025.9
(22) Anmeldetag: 13.06.2008
(51) Int. Cl.: C07D 231/12, C07D 231/14

(54) **VERFAHREN ZUR HERSTELLUNG DIFLUORMETHYLSUBSTITUIERTER PYRAZOLVERBINDUNGEN**
METHOD FOR PRODUCING DIFLUOROMETHYL-SUBSTITUTED PYRAZOLE COMPOUNDS
PROCÉDÉ DE PRÉPARATION DE COMPOSÉS DE PYRAZOLE SUBSTITUÉS PAR DIFLUOROMÉTHYLE

(30) Priorität: 15.06.2007 EP 07110397
(43) Veröffentlichungstag der Anmeldung: 03.03.2010
(73) Patentinhaber: BASF SE, 67056 Ludwigshafen (DE)
(72) Erfinder: NETT, Markus, 67105 Schifferstadt (DE); GROTE, Thomas, 67157 Wachenheim (DE); LOHMANN, Jan Klaas, 68159 Mannheim (DE); DIETZ, Jochen, 76227 Karlsruhe (DE); SMIDT, Sebastian Peer, 68723 Oftersheim (DE); RACK, Michael, 69214 Eppelheim (DE); ZIERKE, Thomas, 67459 Böhl-Iggelheim (DE)
(86) Internationale Anmeldenummer: PCT/EP2008/057506
(87) Internationale Veröffentlichungsnummer: WO 2008/152138

(56) Entgegenhaltungen:
- WO-A-2005/042468
- WO-A-2005/044804
- WO-A-2008/022777
- DE-A1- 10 331 496
- JP-A- 59 046 273
- US-A- 5 093 347
- PETROV V A ET AL: "1,1,2,2-Tetrafluoroethyl-N,N-dimethylamin e: a new selective fluorinating agent" JOURNAL OF FLUORINE CHEMISTRY, ELSEVIER, NL, Bd. 109, Nr. 1, 1. Juni 2001 (2001-06-01), Seiten 25-31, XP004246492 ISSN: 0022-1139
- NATHALIE MERCERON ET AL: "c-Phosphanyl-C-chloroiminium salts as electrophilic carbene synthetic equivalents" CHEMICAL COMMUNICATIONS - CHEMCOM, ROYAL SOCIETY OF CHEMISTRY, GB, 1. Januar 2002 (2002-01-01), Seiten 2250-2251, XP002459062 ISSN: 1359-7345
- GUPTON J T ET AL: "The application of vinylogous iminium salt derivatives to the regiocontrolled preparation of heterocyclic appended pyrazoles" TETRAHEDRON, ELSEVIER SCIENCE PUBLISHERS, AMSTERDAM, NL, Bd. 58, Nr. 27, 1. Juli 2002 (2002-07-01), Seiten 5467-5474, XP004367674 ISSN: 0040-4020

## Beschreibung

Die vorliegende Erfindung betrifft ein Verfahren zur Herstellung 3-difluormethylsubstituierter Pyrazolverbindungen der Formel (I) 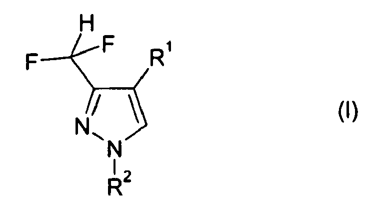 worin
- R¹: für Wasserstoff, C₁-C₈-Alkyl, Phenyl, Cyano oder -C(=O)-OR^{1a} steht, wobei die Grupp Phenyl unsubstituiert ist oder 1 oder 2 Substituenten unabhängig voneinander ausgewählt unter Halogen, Nitro, C₁-C₄-Alkyl, C₁-C₄-Haloalkyl oder C₁-C₄-Alkoxy aufweist, wobei
R^{1a} für C₁-C₈-Alkyl, C₁-C₈-Haloalkyl, C₃-C₈-Cycloalkyl, C₁-C₄-Alkoxy-C₁-C₄-alkyl, C₃-C₈-Cycloalkoxy-C₁-C₄-alkyl, C₂-C₈-Alkenyl, Benzyl oder Phenyl stehen, wobei die Phenylgruppe in Ben- zyl und Phenyl jeweils unsubstituiert ist oder 1, 2 oder 3 Substituenten un- abhängig voneinander ausgewählt unter Halogen, CN, Nitro, C₁-C₄-Alkyl, C₁-C₄-Haloalkyl, C₁-C₄-Alkoxy und C₁-C₄-Haloalkoxy aufweist,
- R²: für Methyl steht.
sowie ein Verfahren zur Überführung solcher Verbindungen in die entsprechenden 3-Difluormethylpyrazol-4-ylcarbonsäuren der Formel (VI) 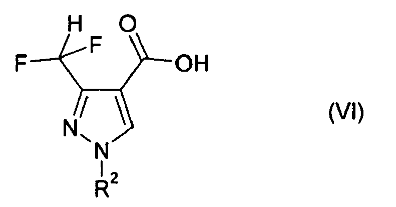 worin R² hier und im Folgenden für methyl steht.

Die WO 92/12970 beschreibt (3-Difluormethyl-1-methylpyrazol-4-yl)carboxamide und deren Verwendung als Fungizide. Die Herstellung dieser Verbindungen erfolgt ausgehend von einem 4,4-Difluoracetessigester, der sukzessive mit Triethylorthoformiat und mit Methylhydrazin umgesetzt wird, wobei man den (3-Difluormethyl-1-methylpyrazol-4-yl)carbonsäureester erhält. Dieser wird anschließend zur Carbonsäure verseift.

Die WO 2005/044804 beschreibt Carbonsäureester fluormethylsubstituierter Heterocyclen, wie unter anderem 3-(Difluormethyl)-1-methyl-1H-pyrazol-4-carbonsäureethylester, sowie deren Herstellung durch Halogenaustausch an den entsprechenden Carbonsäureestern chlormethylsubstituierter Heterocyclen.

Die bislang aus dem Stand der Technik bekannten Verfahren zur Herstellung 3-difluormethylsubstituierter Pyrazolverbindungen gehen entweder von Ausgangsverbindungen aus, deren Bereitstellung vergleichsweise aufwendig oder teuer ist oder verwenden zur Einführung der Difluormethylgruppe Reagenzien auf Fluorwasserstoff- oder Fluorid-basis, die teilweise toxikologisch bedenklich sind und aufgrund ihrer Korrosivität eine großtechnische Umsetzung erschweren. Des Weiteren handelt es sich bei den im Stand der Technik beschriebenen Verfahren um mehrstufige Verfahren, mit einer Vielzahl von Aufarbeitungen und Aufreinigungen der durchlaufenen Zwischenprodukte und damit verbundenen Ausbeuteverlusten.

Der vorliegenden Erfindung liegt somit die Aufgabe zugrunde, ein Verfahren zur Herstellung 3-difluormethylsubstituierter Pyrazolverbindungen bereitzustellen, das von Ausgangsverbindungen ausgeht, die im großtechnischen Maßstab verfügbar sind oder deren Ausgangsverbindungen sich leicht aus großtechnisch verfügbaren Produkten herstellen lassen. Das Verfahren sollte die mit der Aufarbeitung und Aufreinigung von Zwischenprodukten verbundenen Ausbeuteverluste minimieren. Weiterhin sollte der Einsatz korrosiver Fluorreagenzien vermieden werden.

Überraschenderweise wurde gefunden, dass diese Aufgabe durch ein Verfahren gelöst wird, bei dem ein Reaktionsprodukt (II), welches durch Umsetzung eines 1-Amino-1,1,2,2-tetrafluorethans der im Folgenden definierten Formel (III) mit einer Säure und anschließender Umsetzung des erhaltenen Zwischenproduktes mit einer Base und einer Ethylenverbindung der im Folgenden definierten Formel (IV) erhalten wird, mit einer Hydrazinverbindung umgesetzt wird.

Gegenstand der vorliegenden Erfindung ist somit ein Verfahren zur Herstellung von Verbindungen der Formel (I), wie zuvor definiert,
umfassend
A) die Umsetzung einer Verbindung der Formel (III), 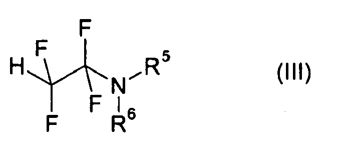 worin
   - R⁵ und R⁶: unabhängig voneinander für C₁-C₄-Alkyl zusammen mit dem Stickstoffatom, an das diese gebunden sind, für einen N-gebundenen 5- bis 6-gliedrigen Heterocyclus stehen, der neben dem Stickstoffatom noch 1 oder 2 weitere Heteroatome ausgewählt unter N, O und S als Ringatome aufweisen kann und unsubstituiert ist oder 1, 2, 3 oder 4 Substituenten, unabhängig voneinander ausgewählt unter Halo- gen, CN, Nitro, C₁-C₄-Alkyl, C₁-C₄-Haloalkyl, C₁-C₄-Alkoxy und C₁-C₄-Haloalkoxy, aufweist;
   mit einer Säure und einer Verbindung der Formel (IV) 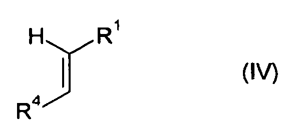 worin
   - R¹: eine der zuvor gegebenen Bedeutungen besitzt und
   - R⁴: für Halogen, -OR^{4a}, -SR^{4a} oder -O-SO₂-R^{4a} steht, worin
   R^{4a} für Wasserstoff, C₁-C₈-Alkyl, C₁-C₈-Haloalkyl, C₂-C₈-Alkenyl, C₃-C₈-Cycloalkyl, Benzyl oder Phenyl steht, wobei die Phenylgruppe in Benzyl und Phenyl jeweils unsubstitu- iert ist oder 1, 2 oder 3 Substituenten unabhängig voneinander ausgewählt unter Halogen, CN, Nitro, C₁-C₄-Alkyl, C₁-C₄-Haloalkyl, C₁-C₄-Alkoxy und C₁-C₄-Haloalkoxy aufweist,
   wobei man ein Reaktionsprodukt (II) erhält, und
B) die Umsetzung des Reaktionsproduktes (II) mit einer Hydrazinverbindung der Formel H₂N-NHR², worin R² eine der zuvor gegebenen Bedeutungen besitzt, unter Erhalt einer Verbindung der Formel (I).

Das erfindungsgemäße Verfahren liefert die Verbindungen der Formel (I) in hohen Ausbeuten bezogen auf die Verbindungen der Formeln (III) und (IV). Der Einsatz korrosiver Reagenzien, wie beispielsweise Reagenzien auf Fluorwasserstoff- oder Fluoridbasis, kann auf diese Weise vermindert werden.

Man nimmt an, dass in Schritt A) des erfindungsgemäßen Verfahrens durch die Umsetzung einer Verbindung der Formel (III) mit einer Säure zunächst durch Abstraktion eines Fluorid-Anions ein reaktives Iminium-lon gebildet wird, das durch Umsetzung mit einer Verbindung der Formel (IV) Verbindungen der Formel (II.a) oder (II.b), 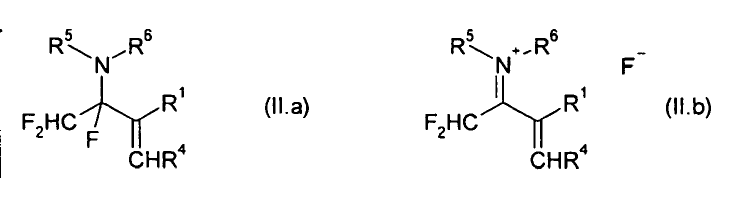 als Reaktionsprodukt (II) bildet, die gegebenenfalls nebeneinander im Gleichgewicht vorliegen. Experimentell konnte das Lewissäure-Addukt der Verbindung (II.b), z.B. mit BF₃ als Lewissäure in Form des Tetrafluororats, nachgewiesen werden. Verbindungen der Formel (II.a) oder (II.b) sowie Lewissäure-Addukte von Verbindungen der Formel (II.b) sind, soweit diese neu sind, ebenfalls Gegenstand der vorliegenden Erfindung.

Die bei der Definition der Variablen verwendeten Begriffe für organische Gruppen sind, wie beispielsweise der Ausdruck "Halogen", Sammelbegriffe, die stellvertretend für die einzelnen Mitglieder dieser Gruppen organischer Einheiten stehen. Das Präfix Cₓ-C_{y} bezeichnet im jeweiligen Fall die Anzahl möglicher Kohlenstoffatome.

Der Begriff "Halogen" bezeichnet jeweils Fluor, Chlor, Brom oder Iod, speziell Fluor, Chlor oder Brom.

Der Begriff "C₁-C₈-Alkyl" bezeichnet eine gesättigte, geradkettige oder verzweigte Kohlenwasserstoffgruppe, umfassend 1 bis 8 Kohlenstoffatome, speziell 1 bis 4 Kohlenstoffatome, beispielsweise Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, 1-Methylhexyl, 2-Methylhexyl, 3-Methylhexyl, 4-Methylhexyl, 5-Methylhexyl, 1,1-Dimethylpentyl, 1,2-Dimethylpentyl, 1,3-Dimethylpentyl, 1,4-Dimethylpentyl, 2,2-Dimethylpentyl, 2,3-Dimethylpentyl, 2,4-Dimethylpentyl, 3,3-Dimethylpentyl, 3,4-Dimethylpentyl, 4,4-Dimethylpentyl, 1,1,2-Trimethylbutyl, 1,1,3-Trimethylbutyl, 1,2,2-Trimethylbutyl, 1,2,3-Trimethylbutyl, 1,3,3-Trimethylbutyl, 2,2,3-Trimethylbutyl, 2,3,3-Trimethylbutyl, 3,3,3-Trimethylbutyl, n-Octyl, 1-Methylheptyl, 2-Methylheptyl, 3-Methylheptyl, 4-Methylheptyl, 5-Methylheptyl, 6-Methylheptyl, 1,1-Dimethylhexyl, 1,2-Dimethylhexyl, 1,3-Dimethylhexyl, 1,4-Dimethylhexyl, 1,5-Dimethylhexyl, 2,2-Dimethylhexyl, 2,3-Dimethylhexyl, 2,4-Dimethylhexyl, 2,5-Dimethylhexyl, 3,3-Dimethylhexyl, 3,4-Dimethylhexyl, 3,5-Dimethylhexyl, 4,4-Dimethylhexyl, 4,5-Dimethylhexyl, 5,5-Dimethylhexyl und deren Isomere. C₁-C₄-Alkyl umfasst beispielsweise Methyl, Ethyl, Propyl, 1-Methylethyl, Butyl, 1-Methylpropyl, 2-Methylpropyl oder 1,1-Dimethylethyl.

Der Begriff "C₁-C₈-Haloalkyl", wie hierin und in den Haloalkyleinheiten von C₁-C₈-Haloalkoxy verwendet, bezeichnet geradkettige oder verzweigte Alkylgruppen mit 1 bis 8 Kohlenstoffatomen, wobei die Wasserstoffatome dieser Gruppen teilweise oder vollständig durch Halogenatome ersetzt sind. C₁-C₄-Haloalkyl steht beispielsweise für Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl oder Pentafluorethyl.

Der Begriff "C₃-C₁₂-Cycloalkyl", vorzugsweise C₃-C₈-Cycloalkyl, bezeichnet mono-, bi- oder tricyclische Kohlenwasserstoffradikale, umfassend 3 bis 12 Kohlenstoffatome, vorzugsweise 3 bis 8 Kohlenstoffatome, speziell 3 bis 6 Kohlenstoffatome. Beispiele monocyclischer Radikale umfassen Cyclopropyl, Cyclobutyl, Cyclopentyl, Cyclohexyl, Cycloheptyl oder Cyclooctyl. Beispiele bicyclischer Radikale umfassen Bicyclo[2.2.1]heptyl, Bicyclo[3.1.1]heptyl, Bicyclo[2.2.2]octyl und Bicyclo[3.2.1]octyl. Beispiele tricyclischer Radikale sind Adamantyl und Homoadamantyl.

Der Begriff "C₂-C₈-Alkenyl", bezeichnet geradkettige und verzweigte ungesättigte Kohlenwasserstoffradikale, umfassend 2 bis 8 Kohlenstoff Atome und wenigstens eine Kohlenstoff-Kohlenstoff-Doppelbindung, wie beispielsweise Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl, 1-Ethyl-2-methyl-2-propenyl, 1-Heptenyl, 2-Heptenyl, 3-Heptenyl, 4-Heptenyl, 5-Heptenyl, 6-Heptenyl, 1-Methyl-1-hexenyl, 1-Methyl-2-hexenyl, 1-Methyl-3-hexenyl, 1-Methyl-4-hexenyl, 1-Methyl-5-hexenyl, 2-Methyl-1-hexenyl, 2-Methyl-2-hexenyl, 2-Methyl-3-hexenyl, 2-Methyl-4-hexenyl, 2-Methyl-5-hexenyl, 3-Methyl-1-hexenyl, 3-Methyl-2-hexenyl, 3-Methyl-3-hexenyl, 3-Methyl-4-hexenyl, 3-Methyl-5-hexenyl, 4-Methyl-1-hexenyl, 4-Methyl-2-hexenyl, 4-Methyl-3-hexenyl, 4-Methyl-4-hexenyl, 4-Methyl-5-hexenyl, 5-Methyl-1-hexenyl, 5-Methyl-2-hexenyl, 5-Methyl-3-hexenyl, 5-Methyl-4-hexenyl, 5-Methyl-5-hexenyl, 1-Octenyl, 2-Octenyl, 3-Octenyl, 4-Octenyl, 5-Octenyl, 6-Octenyl, 7-Octenyl, 1-Methyl-1-heptenyl, 1-Methyl-2-heptenyl, 1-Methyl-3-heptenyl, 1-Methyl-4-heptenyl, 1-Methyl-5-heptenyl, 1-Methyl-6-heptenyl, 2-Methyl-1-heptenyl, 2-Methyl-2-heptenyl, 2-Methyl-3-heptenyl, 2-Methyl-4-heptenyl, 2-Methyl-5-heptenyl, 2-Methyl-6-heptenyl, 3-Methyl-1-heptenyl, 3-Methyl-2-heptenyl, 3-Methyl-3-heptenyl, 3-Methyl-4-heptenyl, 3-Methyl-5-heptenyl, 3-Methyl-6-heptenyl, 4-Methyl-1-heptenyl, 4-Methyl-2-heptenyl, 4-Methyl-3-heptenyl, 4-Methyl-4-heptenyl, 4-Methyl-5-heptenyl, 4-Methyl-6-heptenyl, 5-Methyl-1-heptenyl, 5-Methyl-2-heptenyl, 5-Methyl-3-heptenyl, 5-Methyl-4-heptenyl, 5-Methyl-5-heptenyl, 5-Methyl-6-heptenyl, 6-Methyl-1-heptenyl, 6-Methyl-2-heptenyl, 6-Methyl-3-heptenyl, 6-Methyl-4-heptenyl, 6-Methyl-5-heptenyl, 6-Methyl-6-heptenyl und deren Isomere.

Der Begriff "C₁-C₈-Alkoxy" bezeichnet geradkettige oder verzweigte gesättigte Alkylgruppen, umfassend 1 bis 8 Kohlenstoffatome, die über ein Sauerstoffatom gebunden sind. Beispiele umfassen C₁-C₆-Alkoxy wie beispielsweise Methoxy, Ethoxy, OCH₂-C₂H₅, OCH(CH₃)₂, n-Butoxy, OCH(CH₃)-C₂H₅, OCH₂-CH(CH₃)₂, OC(CH₃)₃, n-Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, n-Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy, 1-Ethyl-2-methylpropoxy, n-Heptyloxy, 1-Methylhexyloxy, 2-Methylhexyloxy, 3-Methylhexyloxy, 4-Methylhexyloxy, 5-Methylhexyloxy, 1,1-Dimethylpentyloxy, 1,2-Dimethylpentyloxy, 1,3-Dimethylpentyloxy, 1,4-Dimethylpentyloxy, 2,2-Dimethylpentyloxy, 2,3-Dimethylpentyloxy, 2,4-Dimethylpentyloxy, 3,3-Dimethylpentyloxy, 3,4-Dimethylpentyloxy, 4,4-Dimethylpentyloxy, 1,1,2-Trimethylbutyloxy, 1,1,3-Trimethylbutyloxy, 1,2,2-Trimethylbutyloxy, 1,2,3-Trimethylbutyloxy, 1,3,3-Trimethylbutyloxy, 2,2,3-Trimethylbutyloxy, 2,3,3-Trimethylbutyloxy, 3,3,3-Trimethylbutyloxy, n-Octyloxy, 1-Methylheptyloxy, 2-Methylheptyloxy, 3-Methylheptyloxy, 4-Methylheptyloxy, 5-Methylheptyloxy, 6-Methylheptyloxy, 1,1-Dimethylhexyloxy, 1,2-Dimethylhexyloxy, 1,3-Dimethylhexyloxy, 1,4-Dimethylhexyloxy, 1,5-Dimethylhexyloxy, 2,2-Dimethylhexyloxy, 2,3-Dimethylhexyloxy, 2,4-Dimethylhexyloxy, 2,5-Dimethylhexyloxy, 3,3-Dimethylhexyloxy, 3,4-Dimethylhexyloxy, 3,5-Dimethylhexyloxy, 4,4-Dimethylhexyloxy, 4,5-Dimethylhexyloxy, 5,5-Dimethylhexyloxy und deren Isomere. C₁-C₄-Alkoxy umfasst beispielsweise Methoxy, Ethoxy, Propoxy, 1-Methylethoxy, Butoxy, 1-Methylpropoxy, 2-Methylpropoxy oder 1,1-Dimethylethoxy.

Der Begriff "C₁-C₄-Alkoxy-C₁-C₄-alkyl" bezeichnet C₁-C₄-Alkyl-Radikale, wobei ein Kohlenstoffatom an ein C₁-C₄-Alkoxy-Radikal gebunden ist. Beispiele hierfür sind CH₂-OCH₃, CH₂-OC₂H₅, n-Propoxymethyl, CH₂-OCH(CH₃)₂, n-Butoxymethyl, (1-Methylpropoxy)methyl, (2-Methylpropoxy)methyl, CH₂-OC(CH₃)₃, 2-(Methoxy)ethyl, 2-(Ethoxy)ethyl, 2-(n-Propoxy)ethyl, 2-(1-Methylethoxy)ethyl, 2-(n-Butoxy)ethyl, 2-(1-Methylpropoxy)ethyl, 2-(2-Methylpropoxy)ethyl, 2-(1,1-Dimethylethoxy)ethyl, 2-(Methoxy)propyl, 2-(Ethoxy)propyl, 2-(n-Propoxy)propyl, 2-(1-Methylethoxy)propyl, 2-(n-Butoxy)propyl, 2-(1-Methylpropoxy)propyl, 2-(2-Methylpropoxy)propyl, 2-(1,1-Dimethylethoxy)propyl, 3-(Methoxy)propyl, 3-(Ethoxy)propyl, 3-(n-Propoxy)propyl, 3-(1-Methylethoxy)propyl, 3-(n-Butoxy)propyl, 3-(1-Methylpropoxy)propyl, 3-(2-Methylpropoxy)propyl, 3-(1,1-Dimethylethoxy)propyl, 2-(Methoxy)butyl, 2-(Ethoxy)butyl, 2-(n-Propoxy)butyl, 2-(1-Methylethoxy)butyl, 2-(n-Butoxy)butyl, 2-(1-Methylpropoxy)butyl, 2-(2-Methylpropoxy)butyl, 2-(1,1-Dimethylethoxy)butyl, 3-(Methoxy)butyl, 3-(Ethoxy)butyl, 3-(n-Propoxy)butyl, 3-(1-Methylethoxy)butyl, 3-(n-Butoxy)butyl, 3-(1-Methylpropoxy)butyl, 3-(2-Methylpropoxy)butyl, 3-(1,1-Dimethylethoxy)butyl, 4-(Methoxy)butyl, 4-(Ethoxy)butyl, 4-(n-Propoxy)butyl, 4-(1-Methylethoxy)butyl, 4-(n-Butoxy)butyl, 4-(1-Methylpropoxy)butyl, 4-(2-Methylpropoxy)butyl oder 4-(1,1-Dimethylethoxy)butyl.

Der Begriff "N-gebundener 5- bis 6-gliedriger Heterocyclus" bezeichnet stickstoffhaltige cyclische Gruppen mit 5 oder 6 Ringatomen, die über ein Stickstoffringatom an den übrigen Teil der Verbindung gebunden sind, wobei der Ring neben dem Stickstoffringatom, über das dieser gebunden ist, gegebenenfalls 1 oder 2 weitere Heteroatome als Ringatome aufweist, die ausgewählt sind unter N, O und S, und wobei der Ring unsubstituiert ist oder 1, 2 oder 3 Substituenten ausgewählt unter Halogen, CN, Nitro, C₁-C₄-Alkyl, C₁-C₄-Haloalkyl, C₁-C₄-Alkoxy und C₁-C₄-Haloalkoxy aufweist.

Beispiele N-gebundener Heterocyclen sind Pyrrolyl, Pyrazolyl, Imidazolyl, Triazolyl, Pyrrolidinyl, Pyrazolinyl, Pyrazolidinyl, Imidazolinyl, Imidazolidinyl, Piperidinyl, Piperazinyl, Morpholinyl, die jeweils über ein Ringstickstoffatom an die restliche Verbindung gebunden sind und unsubstituiert sind oder 1, 2, oder 3 Substituenten ausgewählt unter Halogen, CN, Nitro, C₁-C₄-Alkyl, C₁-C₄-Haloalkyl, C₁-C₄-Alkoxy und C₁-C₄-Haloalkoxy aufweisen.

Im Hinblick auf das efindungsgemäße Verfahren weist wenigstens einer der Reste R¹, R^{1a}, R², R⁴, R^{4a}, R⁵ oder R⁶ bevorzugt eine der im Folgenden gegebenen Bedeutung auf. Besonders bevorzugt weisen alle der zuvor genannten Reste eine der im Folgenden gegebenen Bedeutungen auf.

R^{1a} steht bevorzugt für C₁-C₈-Alkyl oder C₃-C₈-Cycloalkyl.

Erfindungsgemäß steht R⁴ in den Verbindungen der Formel (IV) und folglich in den daraus hergestellten Reaktionsprodukten (II) für Halogen, -OR^{4a}, -SR^{4a} oder -O-SO₂-R^{4a}, worin R^{4a} eine der zuvor gegebenen Bedeutungen besitzt. Besonders bevorzugt steht R⁴ für -OR^{4a}, worin R^{4a} eine der zuvor gegebenen Bedeutungen besitzt.

Bevorzugt steht R^{4a} in den Verbindungen der Formeln (IV) und folglich in den daraus hergestellten Reaktionsprodukten (II) für Wasserstoff, C₁-C₄-Alkyl, C₁-C₄-Haloalkyl oder Phenyl, das unsubstituiert ist oder 1 oder 2 Substituenten ausgewählt unter Halogen, Nitro, C₁-C₄-Alkyl und C₁-C₄-Haloalkyl, C₁-C₄-Alkoxy aufweist;

Bevorzugt stehen R⁵ und R⁶ für Methyl oder Ethyl oder zusammen mit dem Stickstoffatom für Piperidin-1-yl, 4-Methylpiperazin-1-yl oder Morpholin-4-yl.

Bei der Umsetzung eines Reaktionsproduktes (II) mit einer Hydrazinverbindung der Formel R²HN-NH₂ in Schritt B) des erfindungsgemäßen Verfahrens wird man in der Regel so vorgehen, dass man das Reaktionsprodukt (II), gegebenenfalls in einem geeigneten Lösungsmittel oder als Reaktionsgemisch enthaltend das Reaktionsprodukt (II), vorlegt und die Hydrazinverbindung, gegebenenfalls in einem geeigneten Lösungsmittel, hinzugibt.

Alternativ kann man die Hydrazinverbindung, gegebenenfalls in einem geeigneten Lösungsmittel, vorlegen und das Reaktionsprodukt (II), gegebenenfalls in einem geeigneten Lösungsmittel oder als Reaktionsgemisch enthaltend das Reaktionsprodukt (II), hinzugeben.

Üblicherweise wird man in Schritt B) des erfindungsgemäßen Verfahrens die Hydrazinverbindung der Formel R²HN-NH₂ in einer Menge von 0,5 bis 3 Mol, bevorzugt 0,7 bis 1,5 Mol und besonders bevorzugt 0,9 bis 1,2 Mol, bezogen auf ein Mol des Reaktionsproduktes (II) bzw. auf ein Mol der zu dessen Herstellung verwendeten Verbindungen der Formel (III) oder (IV) verwenden.

Vorzugsweise führt man die Umsetzung des Reaktionsproduktes (II) mit einer Hydrazinverbindung der Formel R²HN-NH₂ bei Temperaturen von -80 bis 30 °C und insbesondere bei Temperaturen von - 50 bis 10 °C durch.

Die Hydrazinverbindungen der Formel R²HN-NH₂, können in Schritt B) des erfindungsgemäßen Verfahrens in reiner Form oder in Form ihrer Solvate, z.B. in Form ihrer Hydrate, eingesetzt werden.

Bevorzugt verwendet man die Hydrazinverbindungen der Formel R²HN-NH₂ oder deren Solvat in Schritt B) des erfindungsgemäßen Verfahrens als Lösung in einem geeigneten inerten Lösungsmittel. Bevorzugt wird man eine Lösung der Hydrazinverbindung in dem für die Umsetzung verwendeten Lösungsmittel einsetzen. Geeignete Lösungsmittel sind die im Folgenden Genannten:

Wasser, wässrige Basen, wie Natronlauge oder Kalilauge, oder organische Lösungsmittel, insbesondere Ether, wie Diethylether, Diisopropylether, Methyl-tert-butylether, Tetrahydrofuran, Dioxan oder Anisol, Nitrile, wie Acetonitril oder Propionitril, Ketone, wie Aceton, Methylethylketon, Diethylketon oder Methyl-tert-butylketon, Alkohole, wie Methanol, Ethanol, n-Propanol, Isopropanol, n-Butanol oder tert-Butanol, Dimethylsulfoxid, Dimethylformamid, N-Methylpyrrolidinon (NMP), 1,3-Dimethyl-2-imidazolidinon (DMI), Dimethylacetamid, Dimethylethylenharnstoff, Dimethylpropylenharnstoff (DMPU) oder Tetramethylharnstoff oder Gemische der zuvor genannten Lösungsmittel.

Bevorzugte Lösungsmittel sind Wasser, C₁-C₄-Alkohole, wässrige Basen oder Gemische dieser Lösungsmittel.

Geeignete Hydrazinverbindungen der Formel R²HN-NH₂ sind insbesondere Hydrazin bzw. Hydrazinhydrat, Methylhydrazin, Ethylhydrazin, Phenylhydrazin, Chlorphenylhydrazin, Bromphenylhydrazin, Nitrophenylhydrazin, Dinitrophenylhydrazin, Tolylhydrazin, Benzylhydrazin oder Nitrobenzylhydrazin. Besonders bevorzugt wird in dem erfindungsgemäßen Verfahren Methylhydrazin als Hydrazinverbindungen der Formel R²HN-NH₂ verwendet.

Die Umsetzung einer Verbindung der Formel (III) in Schritt A) des erfindungsgemäßen Verfahrens kann beispielsweise mit einer Broensted-Säure erfolgen. Geeignete Broensted-Säuren sind beispielsweise HF, HCl, HBr, H₂SO₄, Sulfonsäuren, wie Methansulfonsäure, Benzolsulfonsäure, p-Toluolsulfonsäure, Trifluormethansulfonsäure oder Tetrafluorethansulfonsäure, oder fluorierte Carbonsäuren, wie Trifluoressigsäure .

Üblicherweise wird man in Schritt A) des erfindungsgemäßen Verfahrens bei der Umsetzung einer Verbindung der Formel (III) mit einer Säure so vorgehen, dass man die Verbindung der Formel (III), gegebenenfalls in einem geeigneten Lösungsmittel oder als Reaktionsgemisch enthaltend die Verbindung der Formel (III), vorlegt und die Säure, gegebenenfalls in einem geeigneten Lösungsmittel, hinzu gibt.

Üblicherweise wird man in Schritt A) des erfindungsgemäßen Verfahrens die Säure in einer Menge von 0,01 bis 4 Mol, bevorzugt 0,5 bis 3 Mol, besonders bevorzugt 0,8 bis 2,5 Mol und ganz besonders bevorzugt 0,9 bis 2,2 Mol, bezogen auf ein Mol der Verbindung der Formel (III) verwenden.

Vorzugsweise führt man die Umsetzung einer Verbindung der Formel (III) mit einer Säure bei Temperaturen von -80 bis 100 °C und insbesondere bei Temperaturen von -10 bis 30 °C durch.

Bevorzugt wird man die Verbindung der Formel (III) in Schritt A) des erfindungsgemäßen Verfahrens jedoch mit einer Lewis-Säure umsetzen.

Die Umsetzung einer Verbindung der Formel (III) mit einer Lewis-Säure unter Abstraktion eines Fluorid-Anions ist an sich bekannt und wird beispielsweise in Journal of the Chemical Society, Chemical Communications 1975, 956 beschrieben.

Üblicherweise wird man bei der Umsetzung einer Verbindung der Formel (III) mit einer Lewis-Säure in Schritt A) des erfindungsgemäßen Verfahrens so vorgehen, dass man die Verbindung der Formel (III), gegebenenfalls in einem geeigneten Lösungsmittel oder als Reaktionsgemisch enthaltend die Verbindung der Formel (III), vorlegt und die Lewis-Säure, gegebenenfalls in einem geeigneten Lösungsmittel, hinzu gibt.

Üblicherweise wird man in Schritt A) des erfindungsgemäßen Verfahrens die Lewis-Säure in einer Menge von 0,01 bis 4 Mol, bevorzugt 0,5 bis 3 Mol, besonders bevorzugt 0,8 bis 2,5 Mol und ganz besonders bevorzugt 0,9 bis 2,2 Mol, bezogen auf ein Mol der Verbindung der Formel (III) verwenden.

Vorzugsweise führt man die Umsetzung einer Verbindung der Formel (III) mit einer Lewis-Säure bei Temperaturen von -80 bis 100 °C und insbesondere bei Temperaturen von -10 bis 30 °C durch.

Die Lewis-Säuren, können in Schritt A) des erfindungsgemäßen Verfahrens in reiner Form oder in Form ihrer Komplexe, z. B. in Form ihrer Etherate, eingesetzt werden. Geeignete Lewis-Säuren sind beispielsweise Verbindungen der Formeln LiX, MgX₂, CaX₂, BX₃, R^{LS}-BX₂, (R^{LS})₂BX, (R^{LS})₃B, AlX₃, R^{LS}-AlX₂, (R^{LS})₂AlX, (R^{LS})₃Al, ScX₃, TiX₄, R^{LS}OTiX₃, (R^{LS}O)₂TiX₂, (R^{LS}O)₃TiX, (R^{LS}O)₄Ti, ZrX₄, FeX₃, NiX₂, CuX, CuX₂, ZnX₂, SiX₄, R^{LS}OSiX₃, (R^{LS}O)₂SiX₂, (R^{LS}O)₃SiX, SnX₄, GeX₄, PX₅, AsX₅, SbX₅, BiX₃, worin X für Halogen, CN, Trifluormethylsulfonat oder OH steht und R^{LS} für C₁-C₄-Alkyl steht, oder Gemische der zuvor genannten Lewis-Säuren. Bevorzugt steht X für F, Cl oder Trifluormethylsulfonat. R^{LS} steht bevorzugt für Methyl (Me), Ethyl oder Isopropyl. Bevorzugte Lewis-Säuren sind MgF₂, MgCl₂, CaCl₂, BF₃, B(OH)₃, AlCl₃, MeAlCl₂, Me₂AlCl, SiCl₄, Me₃SiCl, TiCl₄ und ZnCl₂. Eine besonders bevorzugte Lewis-Säure ist BF₃. Beispiele bevorzugter Komplexe sind BF₃-Diethyletherat, BF₃-Dimethyletherat, BF₃-Tetrahydrofuranat oder BF₃-Aminkomplexe, wie der BF₃-Pyridinkomplex.

Bevorzugt verwendet man die Lewis-Säure oder deren Solvat in Schritt A) des erfindungsgemäßen Verfahrens als Lösung in einem geeigneten inerten Lösungsmittel. Bevorzugt wird man eine Lösung der Lewis-Säure in dem für die Umsetzung verwendeten Lösungsmittel einsetzen. Bevorzugte Lösungsmittel sind die im Folgenden Genannten.

Geeignete Lösungsmittel für Schritt A) des erfindungsgemäßen Verfahrens sind in der Regel aprotische organische Lösungsmittel. Beispiele sind aromatische Kohlenwasserstoffe, wie Toluol, o-Xylol, m-Xylol oder p-Xylol, halogenierte Kohlenwasserstoffe, wie Methylenchlorid, Chloroform oder Chlorbenzol, Ether, wie Diethylether, Diisopropylether, Methyl-tert-butylether, Cyclopentylmethylether, Tetrahydrofuran, 2-Methyltetrahydrofuran, Dioxan oder Anisol, Nitrile, wie Acetonitril oder Propionitril, Ketone, wie Aceton, Methylethylketon, Diethylketon oder Methyl-tert-butylketon, Dimethylsulfoxid, Dimethylformamid, N-Methylpyrrolidinon (NMP), 1,3-Dimethyl-2-imidazolidinon (DMI), Dimethylacetamid, Dimethylethylenharnstoff, Dimethylpropylenharnstoff (DMPU) oder Tetramethylharnstoff oder Gemische der zuvor genannten Lösungsmittel. Bevorzugte Lösungsmittel sind Ether, insbesondere Diethylether, Tetrahydrofuran, Dioxan sowie deren Gemische.

Vorzugsweise führt man Schritt A) des erfindungsgemäßen Verfahrens in weitgehender Abwesenheit von Wasser, d. h. in einem trockenen organischen Lösungsmittel, durch. Hier und im Folgenden bedeutet "trocken", dass das Lösungsmittel einen Wassergehalt von höchstens 500 ppm und insbesondere von höchstens 100 ppm aufweist. Zudem kann es vorteilhaft sein, zum Ausschluss von Wasser Schritt A) des erfindungsgemäßen Verfahrens unter Schutzgasatmosphäre, beispielsweise unter Stickstoffatmosphäre, durchzuführen.

Man nimmt an, dass bei der Umsetzung einer Verbindung der Formel (III) mit einer Säure in Schritt A) des erfindungsgemäßen Verfahrens durch Abstraktion eines Fluorid-Anions ein reaktives Iminium-Ion der folgenden Formel, 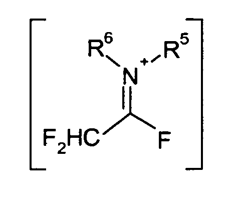 gebildet wird. Dieses reaktive Umsetzungsprodukt wird bevorzugt ohne vorherige Isolierung in dem darauf folgenden Umsetzungsschritt eingesetzt.

In einer bevorzugten Ausführungsform des erfindungsgemäßen Verfahrens wird daher das gemäß dem erfindungsgemäßen Verfahren bei der Umsetzung einer Verbindung der Formel (III) mit einer Säure erhaltene Reaktionsgemisch, enthaltend ein reaktives Umsetzungsprodukt, ohne vorherige Isolierung mit einer Verbindung der Formel (IV) umgesetzt.

Üblicherweise wird man das bei der Umsetzung einer Verbindung der Formel (III) mit einer Säure erhaltene Produkt, gegebenenfalls als Reaktionsgemisch vorlegen und die Verbindung der Formel (IV), gegebenenfalls in einem geeigneten Lösungsmittel, hinzugeben.

Üblicherweise wird man in Schritt A) des erfindungsgemäßen Verfahrens die Verbindung der Formel (IV) in einer Menge von 0,5 bis 3 Mol, bevorzugt 0,7 bis 2 Mol und besonders bevorzugt 0,8 bis 1,2 Mol, bezogen auf ein Mol des Reaktionsproduktes (II) oder bezogen auf ein Mol der eingesetzten Verbindung der Formel (III) verwenden. Bezüglich der Gesamtausbeute des erfindungsgemäßen Verfahrens hat es sich als besonders vorteilhaft erwiesen, die Verbindung der Formel (IV) im Unterschuss bezogen auf die Verbindung der Formel (III) einzusetzen, d.h. weniger als 1 Mol der Verbindung der Formel (IV) bezogen auf ein Mol der Verbindung (III).

Vorzugsweise führt man die Umsetzung des durch Umsetzung einer Verbindung der Formel (III) mit einer Säure erhaltenen Produktes mit einer Verbindung der Formel (IV) in Schritt A) des erfindungsgemäßen Verfahrens bei Temperaturen von -10 bis 100 °C und insbesondere bei Temperaturen von 0 bis 40 °C durch.

Insbesondere eignen sich Verbindungen der Formel (IV), in denen R¹ für einen verseifbaren Rest CN oder C(=O)-OR^{1a} und insbesondere für einen Rest C(=O)-OR^{1a} steht, worin R^{1a} eine der zuvor gegebenen Bedeutungen aufweist. Ebenfalls geeignet sind Verbindungen der Formel (IV), worin R¹ für einen inerten Rest, z. B. Wasserstoff, C₁-C₈-Alkyl oder gegebenenfalls substituiertes Phenyl, und insbesondere für Wasserstoff steht.

In einer speziellen Ausführungsform des erfindungsgemäßen Verfahrens wird die Umsetzung mit einer Verbindung der Formel (IV) ohne Zusatz einer von der Verbindung der Formel (III) bzw. deren Umsetzungsprodukt verschiedenen Base durchführen.

In einer weiteren speziellen Ausführungsform des erfindungsgemäßen Verfahrens wird die Umsetzung mit einer Verbindung der Formel (IV) zusätzlich in Gegenwart einer geeigneten Base durchgeführt. Die Zugabe der Base erfolgt dabei bevorzugt vor oder zeitgleich mit der Zugabe der Verbindung der Formel (IV).

Als zusätzlich verwendete Basen in Schritt A) des erfindungsgemäßen Verfahrens eignen sich organische Basen im Allgemeinen. Geeignete Basen sind insbesondere tertiäre Amine, beispielsweise Tri(C₁-C₆-alkyl)amine, wie Trimethylamin, Triethylamin oder Di-isopropylethylamin, cyclische Amine, wie N-Methylpiperidin, aromatische Amine, wie Pyridin, 2,4,6-Trimethylpyridin (Collidin), 2,6-Dimethylpyridin (Lutidin), 2-Methylpyridin (2-Picolin), 3-Methylpyridin (3-Picolin) oder 4-Dimethylaminopyridin, sowie bicyclische Amine, wie 1,4-Diazabicyclo[2.2.2]octan, 1,8-Diazabicyclo[5.4.0]undec-7-en oder 1,5-Diazabicyclo[4.3.0]non-5-ene. Bevorzugt werden aromatische Amine, besonders bevorzugt Pyridin, Picolin, Lutidin oder Collidin verwendet.

Die Basen werden im Allgemeinen in einer Menge von 0,9 bis 2 Mol, bezogen auf 1 Mol der Verbindungen der Formel (III), vorzugsweise in einer Menge von 0,95 bis 1,5 Mol, bezogen auf 1 Mol der Verbindungen der Formel (III), eingesetzt. Sie können grundsätzlich aber auch in einem größeren Überschuss eingesetzt werden.

Vorteilhafterweise lässt sich das in Schritt A) des erfindungsgemäßen Verfahrens erhaltene Reaktionsgemisch, enthaltend ein Reaktionsprodukt (II), ohne vorherige Aufarbeitung in Schritt B) des Verfahrens einsetzen. In einer speziellen Ausführungsform des erfindungsgemäßen Verfahrens wird demzufolge das in Schritt A) des erfindungsgemäßen Verfahrens erhaltene Reaktionsprodukt (II) ohne vorherige Isolierung mit der Hydrazinverbindung der Formel H₂N-NHR² umgesetzt.

In einer bevorzugten Ausführungsform des erfindungsgemäßen Verfahrens wird das durch Umsetzung einer Verbindung der Formel (III) mit einer Säure erhaltenen Produkt sowie das daraus durch Umsetzung mit einer Verbindung der Formel (IV) hergestellte Reaktionsprodukt (II) ohne vorherige Isolierung in dem Verfahren zur Herstellung von Verbindungen der Formel (I) eingesetzt.

Verbindungen der Formel (III) können beispielsweise durch Umsetzung eines sekundären Amins der Formel (V), 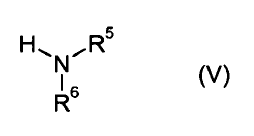 worin R⁵ und R⁶ eine der zuvor gegebenen Bedeutungen besitzen, mit Tetrafluorethylen unter Erhalt einer Verbindung der Formel (III) bereitgestellt werden.

Die Umsetzung von 1,1,2,2-Tetrafluorethylen mit sekundären Aminen ist an sich bekannt und wird beispielsweise in J. Fluorine Chem. 2001, 109, S. 25-31 oder J. Am. Chem. Soc. 1960, 82, 5116 beschrieben.

Üblicherweise wird man bei der Umsetzung von 1,1,2,2-Tetrafluorethylen mit einem sekundären Amin so vorgehen, dass man das sekundäre Amin vorlegt und 1,1,2,2-Tetrafluorethylen hinzu gibt. Die Umsetzung wird bevorzugt in Substanz, d. h. ohne Zusatz eines Lösungsmittels durchgeführt. Bei der Herstellung der Verbindung der Formel (III) und der weiterer Verwendung sind geeignete Maßnahmen zum Schutz vor der Zersetzung der Verbindung der Formel (III) durch Wasser zu treffen.

Üblicherweise wird man bei der Umsetzung von 1,1,2,2-Tetrafluorethylen mit einem sekundären Amin 1,1,2,2-Tetrafluorethylen in einer Menge von 0,5 bis 2 Mol, bevorzugt 0,8 bis 1,2 Mol und besonders bevorzugt 0,9 bis 1,1 Mol, bezogen auf ein Mol des eingesetzten sekundären Amins verwenden.

Vorzugsweise führt man die Umsetzung von 1,1,2,2-Tetrafluorethylen mit einem sekundären Amin bei Temperaturen von -20 bis 60 °C und insbesondere bei Temperaturen von -10 bis 30 °C durch.

Bevorzugte sekundäre Amine für die Umsetzung mit 1,1,2,2-Tetrafluorethylen sind beispielsweise Di-(C₁-C₄-alkyl)amine, wie Dimethylamin, Diethylamin oder Diisopropylamin.

Vorteilhafterweise lässt sich das bei der Umsetzung des sekundären Amins mit 1,1,2,2-Tetrafluorethylen erhaltene Reaktionsgemisch, enthaltend die Verbindungen der Formel (III), ohne vorherige Aufarbeitung in Schritt A) des erfindungsgemäßen Verfahrens einsetzen. In einer bevorzugten Ausführungsform des erfindungsgemäßen Verfahrens werden demzufolge die bei der Umsetzung des sekundären Amins mit 1,1,2,2-Tetrafluorethylen erhaltenen Verbindungen der Formel (III) ohne vorherige Isolierung mit einer Säure umgesetzt.

In einer speziellen Ausführungsform des erfindungsgemäßen Verfahrens sind die Reste R¹ ausgewählt unter Gruppen, die durch Hydrolyse in eine Carboxylgruppe überführbar sind. Diese hydrolisierbaren Reste R¹ werden im Folgenden als Reste R^{1'} bezeichnet. Die Reste R^{1'} sind ausgewählt unter CN, -C(=O)-OR^{1aa}, -C(=O)-NR^{1bb}R^{1cc}, -C(=O)-SR^{1dd} und -C(=S)-SR^{1ee}, wobei R^{1aa}, R^{1bb}, R^{1cc}, R^{1dd} und R^{1ee} eine der zuvor für die entsprechenden Reste R^{1a}, R^{1b}, R^{1c}, R^{1d} und R^{1e} gegebenen Bedeutungen besitzen.

Verbindungen der Formel (I), worin R¹ für einen hydrolisierbaren Rest steht, also R¹ eine der für R^{1'} gegebenen Bedeutungen besitzt, lassen sich durch Hydrolyse in die entsprechenden 3-Difluormethylpyrazol-4-carbonsäuren überführen.

Demzufolge betrifft ein weiterer Gegenstand der vorliegenden Erfindung ein Verfahren zur Herstellung von Verbindungen der Formel (VI), wie zuvor definiert,
umfassend
i) die Herstellung einer Verbindung der Formel (I.a) nach einem der zuvor für die Herstellung von Verbindungen der Formel (I) beschriebenen erfindungsgemäßen Verfahren, 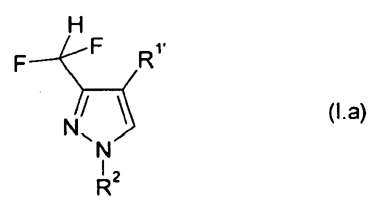 worin
   - R^{1'}: für CN oder -C(=O)-OR^{1aa} steht, wobei R^{1aa} für C₁-C₈-Alkyl, C₁-C₈-Haloalkyl, C₃-C₈-Cycloalkyl, C₁-C₄-Alkoxy-C₁-C₄-alkyl, C₃-C₈-Cycloalkoxy-C₁-C₄-alkyl, C₂-C₈-Alkenyl, Benzyl oder Phenyl steht, wobei die Phenylgruppe in Benzyl und Phenyl jeweils unsubstituiert ist oder 1, 2 oder 3 Substituenten unabhängig voneinander ausgewählt unter Halogen, CN, Nitro, C₁-C₄-Alkyl, C₁-C₄-Haloalkyl C₁-C₄-Alkoxy und C₁-C₄ Haloalkoxy aufweist, und
   - R²: eine der zuvor gegebenen Bedeutungen besitzt; und
ii) die Hydrolyse einer Verbindung der Formel (I.a) unter Erhalt einer Verbindung der Formel (VI).

Die Hydrolyse kann sauer, basisch oder in sonstiger Weise durchgeführt werden. Die Verbindung der Formel (I) kann als solche, d. h. nach Isolierung eingesetzt werden. Es ist jedoch auch möglich das in Schritt B) des erfindungsgemäßen Verfahrens zur Herstellung von Verbindungen der Formel (I) erhaltene Reaktionsgemisch, gegebenenfalls nach Abtrennung flüchtiger Bestandteile wie Lösungsmitteln, ohne weitere Reinigung zur Hydrolyse einzusetzen.

Zur basischen Hydrolyse der Verbindung der Formel (I) wird man üblicherweise die Verbindung der Formel (I) mit einem Alkalimetallhydroxid, wie Natriumhydroxid, Kaliumhydroxid oder Lithiumhydroxid, bevorzugt mit einer wässrigen Lösung eines Alkalimetallhydroxids, speziell mit Natronlauge oder Kalilauge, bis zur vollständigen Hydrolyse des Esters behandeln. Ebenso bevorzugt sind Lösungen von Alkalimetallhydroxid in C₁-C₄-Alkanolen, speziell in Methanol.

Bei der basischen Hydrolyse liegt das Molverhältnis von Verbindung der Formel (I) zu Base üblicherweise im Bereich von 1 : 0,8 bis 1 : 10 und ist insbesondere etwa equimolar (d. h. es liegt im Bereich von 0,9 : 1 bis 1,2 : 1), jedoch kann auch ein größerer Basenüberschuss, z. B. bis zu 5 Mol der Base je Mol der Verbindung der Formel (I), von Vorteil sein.

Üblicherweise erfolgt die basische Hydrolyse in einem Verdünnungs- bzw. Lösungsmittel. Geeignete Verdünnungs- bzw. Lösungsmittel sind neben Wasser auch organische Lösungsmittel, die gegenüber Alkali stabil sind, sowie deren Mischungen mit Wasser. Beispiele für Alkali-stabile organische Lösungsmittel sind insbesondere die vorgenannten C₁-C₄-Alkohole sowie die vorgenannten acyclischen und cyclischen Ether. Vorzugsweise führt man die Hydrolyse in wässriger Phase, d. h. in Wasser oder einer Mischung aus Wasser mit einem der vorgenannten organischen Lösungsmittel durch, wobei der Gehalt an organischem Lösungsmittel in der wässrigen Phase in der Regel 30 Vol.-%, bezogen auf die Gesamtmenge an Wasser und organischem Lösungsmittel typischerweise nicht überschreitet.

Bevorzugt führt man die basische Hydrolyse bei Temperaturen von 0 bis 80 °C, besonders bevorzugt bei 10 bis 60 °C durch. In der Regel ist die obere Temperaturgrenze der Siedepunkt des verwendeten Lösungsmittels bei druckloser Reaktionsführung. Die Reaktionsdauer ist hierbei von der Reaktionstemperatur, der Konzentration und der Stabilität der jeweiligen Esterbindung abhängig. Im Allgemeinen werden die Reaktionsbedingungen so gewählt, dass die Reaktionszeit im Bereich von 1 bis 12 h, insbesondere im Bereich von 2 bis 8 h, liegt.

Die saure Hydrolyse einer Verbindung der Formel (I) kann in Analogie zu bekannten sauren Esterhydrolysen durchgeführt werden, d. h. in Gegenwart katalytischer oder stöchiometrischer Mengen einer Säure und Wasser (siehe z. B. J. March, Advanced Organic Chemistry, 2nd Ed., 334-338, McGraw-Hill, 1977 und dort zitierte Literatur). Häufig wird man die Umsetzung in einer Mischung aus Wasser und einem aprotischen organischen Lösungsmittel, beispielsweise einem Ether, durchführen. Beispiele für geeignete Säuren sind Halogenwasserstoffsäuren, Schwefelsäure, organische Sulfonsäuren, wie p-Toluolsulfonsäure, Methansulfonsäure, Phosphorsäure sowie saure lonentauscherharze und dergleichen.

Geeignete Hydrolysekatalysatoren sind weiterhin Alkalimetalliodide, wie Lithiumiodid, Natriumiodid oder Kaliumiodid, Trimethyliodsilan oder Mischungen von Trimethylchlorsilan mit Alkalimetalliodiden.

Die Isolierung der Verbindung der Formel (VI) erfolgt durch übliche Trennverfahren, wie beispielsweise Fällung durch Einstellen des pH-Werts oder durch Extraktion.

In einer bevorzugten Ausführungsform des Verfahrens zur Herstellung einer Verbindung der Formel (VI) wird die in Schritt B) des erfindungsgemäßen Verfahrens zur Herstellung einer Verbindung der Formel (I) hergestellte Verbindung der Formel (I.a) ohne vorherige Isolierung in Schritt ii) des erfindungsgemäßen eingesetzt.

Bevorzugt steht R^{1aa} für C₁-C₆-Alkyl, wie Methyl oder Ethyl.

Bezüglich des bevorzugten Restes R² gilt das zuvor im Rahmen des erfindungsgemäßen Verfahrens zur Herstellung von Verbindungen der Formel (I) Gesagte entsprechend.

Die durch Hydrolyse erhaltenen Verbindungen der Formel (VI) eignen sich vorteilhaft zur Herstellung einer Vielzahl pharmazeutischer und phytosanitärer Wirkstoffe, beispielsweise zur Herstellung von 3-Difluormethylpyrazol-4-carboxamiden, wie sie u. a. in EP 0589301, WO 03/070705, WO 03/074491, WO 05123690 oder WO 06/087343 beschrieben werden.

Geeignete Methoden zur Herstellung von Carboxamiden durch Umsetzung von Carbonsäuren und deren Derivaten mit Aminen sind dem Fachmann bekannt, z.B. aus dem eingangs zitierten Stand der Technik sowie aus J. March, Advanced Organic Chemistry, 2nd Ed., 382 f, McGraw-Hill, 1977 und Organikum, 21. Auflage Wiley-VCH, Weinheim 2001, S. 481-484 und dort zitierte Literatur.

Beispiele für 3-Difluormethylpyrazol-4-carboxamide, deren Ausgangsverbindungen der Formeln (I) bzw. (VI) sich durch die zuvor beschriebenen erfindungsgemäßen Verfahren herstellen lassen, sind:
N-(2-Bicyclopropyl-2-yl-phenyl)-3-difluormethyl-1-methylpyrazol-4-yl-carboxamid,
N-(3',4',5'-Trifluorbiphenyl-2-yl)-3-d ifl uormethyl-1-methyl-pyrazol-4-yl-carboxamid,
N-(2',4',5'-Trifluorbiphenyl-2-yl)-3-difluormethyl-1-methyl-pyrazol-4-yl-carboxamid,
N-(3',4'-Dichlor-3-fluorbiphenyl-2-yl)-1-methyl-3-difluormethyl-1H-pyrazol-4-yl-carboxamid, N-(3',4'-Difluor-3-fluorbiphenyl-2-yl)-1-methyl-3-difluormethyl-1H-pyrazol-4-yl-carboxamid, N-(3'-Chlor-4'-fluor-3-fluorbiphenyl-2-yl)-1-methyl-3-difluormethyl-1H-pyrazol-4-yl-carboxamid, N-(3',4'-Dichlor-4-fluorbiphenyl-2-yl)-1-methyl-3-difluormethyl-1H-pyrazol-4-yl-carboxamid, N-(3',4'-Difluor-4-fluorbiphenyl-2-yl)-1-methyl-3-difluormethyl-1H-pyrazol-4-yl-carboxamid, N-(3'-Chlor-4'-fluor-4-fluorbiphenyl-2-yl)-1-methyl-3-difluormethyl-1H-pyrazol-4-yl-carboxamid, N-(3',4'-Dichlor-5-fluorbiphenyl-2-yl)-1-methyl-3-difluormethyl-1H-pyrazol-4-yl-carboxamid, N-(3',4'-Difluor-5-fluorbiphenyl-2-yl)-1-methyl-3-difluormethyl-1H-pyrazole-4-yl-carboxamid, N-(3'-Chlor-4'-fluor-5-fluorbiphenyl-2-yl)-1-methyl-3-difluormethyl-1H-pyrazole-4-yl-carboxamid,
N-[2-(1,1,2,3,3,3-Hexafluorpropoxy)phenyl]-3-difluormethyl-1-methyl-1H-pyrazol-4-yl-carboxamid, N-[4'-(Trifluormethylthio)-biphenyl-2-yl]-3-difluormethyl-1-methyl-1H-pyrazol-4-yl-carboxamid, 3-(Difluormethyl)-1-methyl-N-[1,2,3,4-tetrahydro-9-(1-methylethyl)-1,4-methanonaphthalin-5-yl]-1H-pyrazol-4-yl-carboxamid, N-(3'-Chlor-5-fluorbiphenyl-2-yl)-3-(difluormethyl)-1-methylpyrazol-4-yl-carboxamid, N-(4'-Chlor-5-fluorbiphenyl-2-yl)-3-(difluormethyl)-1-methylpyrazol-4-yl-carboxamid,
N-(4'-Chlorbiphenyl-2-yl)-3-(difluormethyl)-1-methylpyrazol-4-yl-carboxamid,
N-(4'-Brombiphenyl-2-yl)-3-(difluormethyl)-1-methylpyrazol-4-yl-carboxamid,
N-(4'-Jodbiphenyl-2-yl)-3-(difluormethyl)-1-methylpyrazol-4-yl-carboxamid,
N-(3',5'-difluorbiphenyl-2-yl)-3-(difluormethyl)-1-methylpyrazol-4-yl-carboxamid,
N-(2-Chlor-4-fluor-phenyl)- 3-(difluormethyl)-1-methylpyrazol-4-yl-carboxamid,
N-(2-Brom-4-fluor-phenyl)- 3-(difluormethyl)-1-methylpyrazol-4-yl-carboxamid und
N-(2-Jod-4-fluor-phenyl)- 3-(difluormethyl)-1-methylpyrazol-4-yl-carboxamid.

Im Folgenden wird die Herstellung difluormethylsubstituierter Pyrazolderivate anhand von Beispielen erläutert.

### Beispiel 1: Herstellung von 3-Difluormethyl-1,4-dimethylpyrazol aus 1,1,2,2-Tetrafluorethyldimethylamin und Ethylprop-1-enylether

Zu einer Lösung von 1,1,2,2-Tetrafluorethyldimethylamin (3,2 g, 22 mmol) in Diethylether (10 ml) und Dioxan (10 ml) wurde unter Stickstoffatmosphäre bei einer Temperatur von 0 bis 5 °C eine Lösung von BF₃-Etherat (49 % BF₃, 5,6 ml, 44 mmol) getropft. Nach beendeter Zugabe wurde das Reaktionsgemisch 5 min gerührt. Anschließend wurden nacheinander Pyridin (1,7 g, 22 mmol) und eine Lösung von Ethylprop-1-enylether (1,7 g, 20 mmol) in Dioxan (2 ml) bei einer Temperatur von 0 bis 5 °C zu dem Reaktionsgemisch getropft. Nach 6-stündigem Rühren bei Raumtemperatur wurde das Reaktionsgemisch bei einer Temperatur von 0 bis 5 °C zu einem Gemisch aus Natriumhydroxid (4,4 g, 110 mmol) und Methylhydrazin (1,4 g, 30 mmol) in Wasser (75 ml) gegeben und anschließend 3 h bei Raumtemperatur gerührt. Anschließend wurde Wasser (50 ml) zugegeben und mit Methyl-tert-butylether extrahiert. Die erhaltenen organischen Phasen wurden über Natriumsulfat getrocknet, filtriert und unter vermindertem Druck vom Lösungsmittel befreit. 3-Difluormethyl-1,4-dimethylpyrazol wurde in einer Ausbeute von 25 % erhalten.

### Beispiel 2: Herstellung von 3-Difluormethyl-1-methylpyrazol-4-carbonsäure aus 1,1,2,2-Tetrafluorethyldimethylamin und 3-Ethoxyacrylsäureethylester

Zu einer Lösung von 1,1,2,2-Tetrafluorethyldimethylamin (3,2 g, 22 mmol) in Diethylether (10 ml) und Dioxan (10 ml) wurde unter Stickstoffatmosphäre bei einer Temperatur von 0 bis 5 °C eine Lösung von BF₃-Etherat (49 % BF₃, 5,6 ml, 44 mmol) getropft. Nach beendeter Zugabe wurde das Reaktionsgemisch 5 min gerührt. Anschließend wurden nacheinander Pyridin (1,7 g, 22 mmol) und eine Lösung von 3-Ethoxyacrylsäureethylester (2,9 g, 20 mmol) in Dioxan (2 ml) bei einer Temperatur von 0 bis 5 °C zu dem Reaktionsgemisch getropft. Nach 6-stündigem Rühren wurde das Reaktionsgemisch bei einer Temperatur von 0 bis 5 °C zu einem Gemisch aus Natriumhydroxid (4,4 g, 110 mmol) und Methylhydrazin (1,4 g, 30 mmol) in Wasser (75 ml) gegeben und anschließend 1 h bei Raumtemperatur gerührt. Anschließend wurde das Reaktionsgemisch auf 60 °C erwärmt und 0,5 h bei dieser Temperatur gerührt. Das Reaktionsgemisch wurde von flüchtigen Bestandteilen befreit. Der erhaltene Rückstand wurde in Wasser (50 ml) aufgenommen, mit Ethylacetat gewaschen und anschließend mit konz. Salzsäure auf einen pH-Wert von 2 gebracht. Der dabei ausgefallene Feststoff wurde durch Filtration isoliert, mit Wasser gewaschen und am Vakuum bei einer Temperatur von 50 °C getrocknet. Isomerenreine 3-Difluormethyl-1-methylpyrazol-4-carbonsäure wurde in einer Ausbeute von 50 % erhalten.

### Beispiel 3: Herstellung von 3-Difluormethyl-1-methylpyrazol-4-carbonsäure aus 1,1,2,2-Tetrafluorethyldimethylamin und 3-Methoxyacrylsäuremethylester

Zu einer Lösung von 1,1,2,2-Tetrafluorethyldimethylamin (30 g, 207 mmol) in Diethylether (90 ml) und Dioxan (90 ml) wurde unter Stickstoffatmosphäre bei einer Temperatur von 0 bis 5 °C eine Lösung von BF₃-Etherat (49 BF₃, 59,6 ml, 420 mmol) getropft. Nach beendeter Zugabe wurde das Reaktionsgemisch 5 min gerührt. Anschließend wurden nacheinander Pyridin (15,9 g, 201 mmol) und 3-Methoxyacrylsäuremethylester (22,3 g, 186 mmol) bei einer Temperatur von 0 bis 5 °C zu dem Reaktionsgemisch getropft. Nach 6-stündigem Rühren entstand ein schmieriger Feststoff, von dem die überstehende Lösung abdekantiert und verworfen wurde. Der Feststoff wurde dann bei einer Temperatur von 0 bis 5 °C zu einem Gemisch aus Natriumhydroxid (41,4 g, 1,035 mol) und Methylhydrazin (38,6 g einer 35%igen wässrigen Lösung, 288 mmol) in Wasser (665 ml) gegeben und anschließend 1 h bei Raumtemperatur gerührt. Anschließend wurde das Reaktionsgemisch auf 60 °C erwärmt und 0,5 h bei dieser Temperatur gerührt. Das Reaktionsgemisch wurde von flüchtigen Bestandteilen befreit. Der erhaltene Rückstand wurde in Wasser (50 ml) aufgenommen, mit Ethylacetat gewaschen und anschließend mit konz. Salzsäure auf einen pH-Wert von 2 gebracht. Der bei einer Temperatur von 0 °C ausgefallene Feststoff wurde durch Filtration isoliert, mit wenig eiskaltem Wasser gewaschen und unter vermindertem Druck bei einer Temperatur von 40 °C getrocknet. 3-Difluormethyl-1-methylpyrazol-4-carbonsäure wurde als Gemisch mit 5-Difluormethyl-1-methylpyrazol-4-carbonsäure mit einem Verhältnis von 85 : 15 in einer in einer Menge von 10,1 g erhalten.

### Beispiel 4: Herstellung von 3-Difluormethyl-1-methylpyrazol-4-carbonsäure aus 1,1,2,2-Tetrafluorethyldimethylamin und 3-Pyrrolidin-1-ylacrylsäureethylester (nicht erfindungsgemäß)

Zu einer Lösung von 1,1,2,2-Tetrafluorethyldimethylamin (3,2 g, 22 mmol) in Diethylether (10 ml) und Dioxan (10 ml) wurde unter Stickstoffatmosphäre bei einer Temperatur von 0 bis 5 °C eine Lösung von BF₃-Etherat (49 % BF₃, 5,6 ml, 44 mmol) getropft. Nach beendeter Zugabe wurde das Reaktionsgemisch 5 min gerührt. Anschließend wurden nacheinander Pyridin (1,7 g, 22 mmol) und eine Lösung von 3-(Pyrrolidin-1-yl)acrylsäureethylester (2,9 g, 20 mmol) in Dioxan (2 ml) bei einer Temperatur von 0 bis 5 °C zu dem Reaktionsgemisch getropft. Nach 6-stündigem Rühren wurde das Reaktionsgemisch bei einer Temperatur von 0 bis 5 °C zu einem Gemisch aus Natriumhydroxid (4,4 g, 110 mmol) und Methylhydrazin (1,4 g, 30 mmol) in Wasser (75 ml) gegeben und anschließend 3 h bei Raumtemperatur gerührt. Das Reaktionsgemisch wurde von flüchtigen Bestandteilen befreit. Der erhaltene Rückstand wurde in Wasser (50 ml) aufgenommen, mit Ethylacetat gewaschen und anschließend mit konz. Salzsäure auf einen pH-Wert von 2 gebracht. Die wässrige Phase wurde abdekantiert und verworfen. Der erhaltene schleimige Rückstand wurde in einem Gemisch aus Tetrahydrofuran und Methyl-tert-butylether aufgenommen, über Natriumsulfat getrocknet, filtriert und unter vermindertem Druck vom Lösungsmittel befreit. 3-Difluormethyl-1-methylpyrazol-4-carbonsäure wurde als Gemisch mit 5-Difluormethyl-1-methylpyrazol-4-carbonsäure mit einem Verhältnis von 2 : 1 in einer Ausbeute von 60 % erhalten.

### Beispiel 5: Herstellung von 3-Difluormethyl-1-methylpyrazol-4-carbonsäuremethylester aus 1,1,2,2-Tetrafluorethyldimethylamin und 3-Methoxyacrylsäuremethylester

Zu einer Lösung von 1,1,2,2 Tetrafluorethyldimethylamin (96%ig, 46 g, 305 mmol) in Acetonitril (100 ml) wurde bei 25 °C unter Argon BF₃-Etherat (38,9 g, 274 mmol) zugetropft. Anschließend wurde unter Rückflussbedingungen (ca. 70 °C) innerhalb von 1 h eine Lösung von 3-Methoxyacrylsäuremethylester (95%ig, 33,5 g, 274 mmol) in Acetonitril (75 ml) zu dem Reaktionsgemisch getropft. Nach 21 h Rühren unter Rückflussbedingungen wurde das Reaktionsgemisch auf 25 °C abgekühlt. Das erhaltene Reaktionsgemisch wurde bei 0 bis 15 °C innerhalb von 1,5 h in eine Lösung von Methylhydrazin (21 g, 457 mmol) in Acetonitril (48 ml) getropft. Nach 0,5 h Rühren bei 25 °C wurde Wasser (100 ml) zugegeben. Das Reaktionsgemisch wurde einmal mit 150 ml und einmal mit 90 ml Methylenchlorid extrahiert. Die vereinigten organischen Phasen wurden mit Wasser (1 x200 ml) gewaschen. Die erhaltene organische Phase (530 g) enthielt It. GC-Flächen-%-Analyse 3-Difluormethyl-1-methylpyrazol-4-carbonsäuremethylester und 5-Difluormethyl-1-methylpyrazol-4-carbonsäuremethylester in einem Verhältnis von 6,8 : 1. Laut quantitativer HPLC-Analyse enthielt die organische Phase 6,7 Gew-% 3-Difluormethyl-1-methylpyrazol-4-carbonsäuremethylester. Dies entspricht einer Ausbeute von 68 % (bezogen auf 3-Methoxyacrylsäuremethylester).

### Beispiel 6: Herstellung von 3-Difluormethyl-1-methylpyrazol-4-carbonsäure aus 1,1,2,2-Tetrafluorethyldimethylamin und 3-Methoxyacrylsäuremethylester

Schritt A): Zu einer Lösung von 1,1,2,2-Tetrafluorethyldimethylamin (96%ig, 48,1 g, 318 mmol) in Acetonitril (97 g) wurde bei 25 °C unter Argon BF₃-Etherat (38,4 g, 270 mmol) zugetropft. Anschließend wurde unter Rückflußbedingungen (ca 70 °C) innerhalb von 1 h eine Lösung von 3-Methoxyacrylsäuremethylester (95%ig, 33,1 g, 271 mmol) in Acetonitril (61 g) zu dem Reaktionsgemisch getropft. Nach 17,5 h Rühren unter Rückflüßbedingungen wurde das Reaktionsgemisch auf 25 °C abgekühlt.

Zur Kontrolle des Reaktionsverlaufs wurden während dieser Umsetzung Proben des Reaktionsgemisches entnommen und mittels NMR-Spektroskopie untersucht. Das Kation der Verbindung der Formel II.b.6 (bzw. deren Lewis-Säure-Addukt) 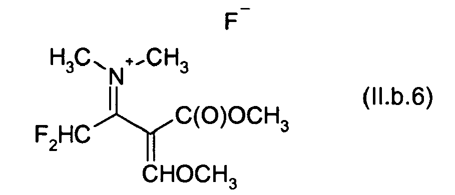 wurden anhand folgender NMR-Verschiebungen im Reaktionsgemisch nachgewiesen:
¹H-NMR (500 MHz, Acetonitril): δ = 7,8 (s, 1H). 6,7 (t, 1 H; ¹J_{HF} 50 Hz), 3,96 (s, 3H), 3,63 (s, 3H,), 3,55 (s, 3H), 3,47 ppm (s, 3H); ¹³C-NMR (125 MHz, Acetonitril): δ = 47, 50, 53, 100, 110, 164, 172 (=CH-OCH₃), 172 ppm (-C=N+(CH₃)₂); ¹⁵N-NMR (500 MHz, Acetonitril, ext. Standard: CH₃NO₂): -167 ppm.

### Schritt B): Alternative 1 (mit wässrigem Methylhydrazin)

Die Hälfte des in Schritt A) erhaltenen Reaktionsgemisches wurde bei 0 bis 15 °C innerhalb von 0,5 h zu einer wässrigen Methylhydrazin-Lösung (30%ig, 38,2 g, 249 mmol) getropft. Nach weiteren 0,5 h Rühren bei 25°C wurde bei 25 bis 30°C eine Lösung von NaOH in Methanol (12,9 Gew.-%ig, 148,3 g, 478 mmol) zu dem Reaktionsgemisch gegeben. Das Reaktionsgemisch wurde weitere 12 h bei 25 °C gerührt. Anschließend wurde das Reaktionsgemisch am Vakuum von flüchtigen Bestandteilen befreit. Der Rückstand wurde in Wasser (130 ml) aufgenommen und mit Toluol (50 ml) gewaschen. Danach wurde der pH-Wert mit Salzsäure (konz., 111 g) auf pH 1 eingestellt. Der dabei ausfallende gelbliche Feststoff wurde abfiltriert, mit Wasser (25 ml) gewaschen und am Vakuum bei 25°C getrocknet. 3-Difluormethyl-1-methylpyrazol-4-carbonsäure wurde als Feststoff erhalten (Ausbeute: 12,6 g; Reinheit laut HPLC: 92 Fl.-% bzw. 70 Gew.-%).

### Schritt B): Alternative 2 (mit wasserfreiem Methylhydrazin)

Die zweite Hälfte des in Schritt A) erhaltenen Reaktionsgemisches wurde bei 0 bis 15 °C innerhalb von 0,5 h zu einer Lösung von Methylhydrazin in Acetonitril (30%ig, 36,6 g, 239 mmol) getropft. Nach weiteren 0,5 h Rühren bei 25°C wurde bei 25 bis 30 °C eine Lösung von NaOH in Methanol (12,9 Gew.-%ig, 148,3 g, 478 mmol) zu dem Reaktionsgemisch gegeben. Das Reaktionsgemisch wurde weitere 12 h bei 25 °C gerührt. Anschließend wurde das Reaktionsgemisch am Vakuum von flüchtigen Bestandteilen befreit. Der Rückstand wurde in Wasser (130 ml) aufgenommen und mit Toluol (50 ml) gewaschen. Danach wurde der pH-Wert mit Salzsäure (konz., 110 g) auf pH 1 eingestellt. Der dabei ausfallende gelbliche Feststoff wurde abfiltriert, mit Wasser (25 ml) gewaschen und am Vakuum bei 25°C getrocknet. 3-Difluormethyl-1-methylpyrazol-4-carbonsäure wurde als Feststoff erhalten (Ausbeute: 13,1 g; Reinheit laut HPLC: 92 Fl.-% bzw. 70 Gew.-%).

### Beispiel 7: Herstellung von 3-Difluormethyl-1H-pyrazol-4-carbonsäuremethylester aus 1,1,2,2-Tetrafluorethyldimethylamin und 3-Methoxyacrytsäuremethylester (nicht erfindungsgemäß)

Zu einer Lösung von 1,1,2,2-Tetrafluorethyldimethylamin (96%ig, 46 g, 305 mmol) in Acetonitril (100 ml) wurde bei 25 °C unter Argon BF₃-Etherat (36,8 g, 259 mmol) zugetropft. Anschließend wurde unter Rückflussbedingungen (ca. 70 °C) innerhalb von 1 h eine Lösung von 3-Methoxyacrylsäuremethylester (95%ig, 31,6 g, 259 mmol) in Acetonitril (75 ml) zu dem Reaktionsgemisch getropft. Nach weiteren 26 h Rühren unter Rückflussbedingungen wurde das Reaktionsgemisch auf 25 °C abgekühlt. Das erhaltene Reaktionsgemisch wurde bei 0 bis 15 °C innerhalb von 1 h zu einer Lösung von Hydrazin-Hydrat (66,5 g, 850 mmol) in Acetonitril (100 ml) getropft. Nach einer weiteren Stunde Rühren bei 25 °C wurde eine Probe der Lösung entnommen und durch HPLC-Analyse untersucht. Das Reaktionsgemisch enthielt lt. HPLC-Analyse als Hauptprodukt (53 Fl.-%) 3-Difluormethyl-1H-pyrazol-4-carbonsäuremethylester (Retentionszeit: 10 min; HPLC-MS: (m/z) = 177).

## Patentansprüche

1. Verfahren zur Herstellung von Verbindungen der Formel (I), 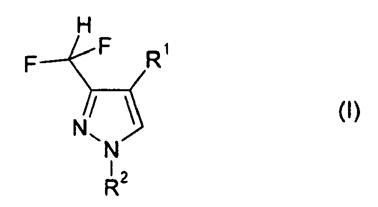 worin
R¹ für Wasserstoff, C₁-C₈-Alkyl, Phenyl, Cyano oder -C(=O)-OR^{1a} steht, wobei die Gruppe Phenyl unsubstituiert ist oder 1 oder 2 Substituenten unabhän- gig voneinander ausgewählt unter Halogen, Nitro, C₁-C₄-Alkyl, C₁-C₄-Haloalkyl und C₁-C₄-Alkoxy aufweist, wobei
R^{1a} für C₁-C₈-Alkyl, C₁-C₈-Haloalkyl, C₃-C₈-Cycloalkyl, C₁-C₄-Alkoxy- C₁-C₄-alkyl, C₃-C₈-Cycloalkoxy-C₁-C₄-alkyl, C₂-C₈-Alkenyl, Benzyl oder Phenyl stehen, wobei die Phenylgruppe in Benzyl und Phenyl jeweils unsubstituiert ist oder 1, 2 oder 3 Substituenten unabhängig voneinander ausgewählt unter Halogen, CN, Nitro, C₁-C₄-Alkyl, C₁-C₄-Haloalkyl, C₁-C₄-Alkoxy und C₁-C₄-Haloalkoxy aufweist; und
R² für Methyl steht;
umfassend
A) die Umsetzung einer Verbindung der Formel (III) 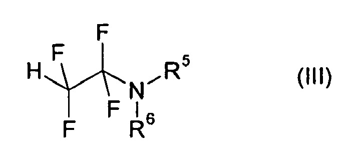 worin
R⁵ und R⁶ unabhängig voneinander für C₁-C₄-Alkyl oder zusammen mit dem Stickstoffatom, an das diese gebunden sind, für einen N- gebundenen 5- bis 6-gliedrigen Heterocyclus stehender neben dem Stickstoffatom noch 1 oder 2 weitere Heteroatome ausgewählt unter N, O und S als Ringatome aufweisen kann und unsubstituiert ist oder 1, 2, 3 oder 4 Substituenten unabhängig voneinander ausgewählt un- ter Halogen, CN, Nitro, C₁-C₄-Alkyl, C₁-C₄-Haloalkyl, C₁-C₄-Alkoxy und C₁-C₄-Haloalkoxy aufweist;
mit einer Säure und einer Verbindung der Formel (IV) 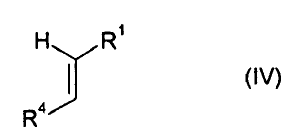 worin
R¹ eine der zuvor gegebenen Bedeutungen besitzt und
R⁴ für Halogen, -OR^{4a}, -SR^{4a} oder -O-SO₂-R^{4a}, worin
R^{4a} für Wasserstoff, C₁-C₈-Alkyl, C₁-C₈-Haloalkyl, C₂-C₈-Alkenyl, C₃-C₈-Cycloalkyl, Benzyl oder Phenyl steht, wobei die Phenylgruppe in Benzyl und Phenyl jeweils unsubstituiert ist oder 1, 2 oder 3 Substi- tuenten unabhängig voneinander ausgewählt unter Halogen, CN, Nitro, C₁-C₄-Alkyl, C₁-C₄-Haloalkyl, C₁-C₄-Alkoxy und C₁-C₄-Halo- alkoxy aufweist,
wobei man ein Reaktionsprodukt (II) erhält, und
B) die Umsetzung des Reaktionsproduktes (II) mit einer Hydrazinverbindung der Formel H₂N-NHR², worin R² eine der zuvor gegebenen Bedeutungen besitzt, unter Erhalt einer Verbindung der Formel (I).

2. Verfahren nach Anspruch 1, wobei man das in Schritt A hergestellte Reaktionsprodukt (II) ohne vorherige Isolierung mit der Hydrazinverbindung der Formel H₂N-NHR² zur Herstellung von Verbindungen der Formel (I) umsetzt.

3. Verfahren nach einem der Ansprüche vorhergehenden Ansprüche, wobei die zur Umsetzung der Verbindung der Formel (III) eingesetzte Säure eine Lewissäure ist.

4. Verfahren nach Anspruch 1, worin R⁴ für -OR^{4a} steht, worin R^{4a} eine der zuvor gegebenen Bedeutungen besitzt.

5. Verfahren zur Herstellung von Verbindungen der Formel (VI), 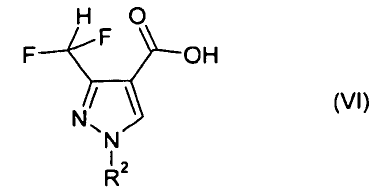 worin
R² für Methyl steht;
umfassend
i) die Herstellung einer Verbindung der Formel (I.a) gemäß einem der zuvor für die Herstellung von Verbindungen der Formel (I) genannten erfindungsgemäßen Verfahren, 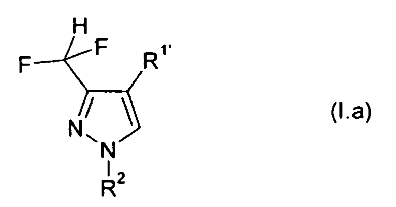 worin
R^{1'} für CN oder -C(=O)-OR^{1aa} steht, wobei R^{1aa} für C₁-C₈-Alkyl, C₁-C₈-Haloalkyl, C₃-C₈-Cycloalkyl, C₁-C₄-Alkoxy-C₁-C₄-alkyl, C₃-C₈-Cycloalkoxy-C₁-C₄-alkyl, C₂-C₈-Alkenyl Benzyl oder Phenyl steht, wo- bei die Phenylgruppe in Benzyl und Phenyl jeweils unsubstituiert ist oder 1, 2 oder 3 Substituenten unabhängig voneinander ausgewählt unter Halogen, (N) Nitro, C₁-C₄- alkyl, C₁-C₄-Haloalkyl C₁-C₄-Alkoxy aufweist und C₁-C₄ Haloalkoxy und
R² eine der zuvor gegebenen Bedeutungen besitzt; und
ii) die Hydrolyse einer Verbindung der Formel (I.a) unter Erhalt einer Verbindung der Formel (VI).

6. Verfahren nach Anspruch 5, worin die in Schritt i) hergestellte Verbindung der Formel (I.a) ohne vorherige Isolierung in Schritt ii) des erfindungsgemäßen Verfahrens zur Herstellung einer Verbindung der Formel (VI) eingesetzt wird.

7. Verwendung von Verbindungen der Formel (III), wie in Anspruch 1 definiert in einem Verfahren gemäß einem der Ansprüche 1 bis 6 zur Herstellung von Verbindungen der Formeln (I) oder (VI).

8. Verbindungen der Formeln (II.a) oder (II.b), 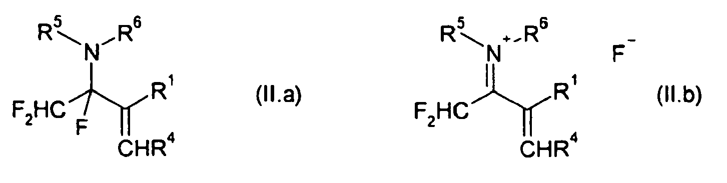 worin R¹ R⁴, R⁵ und R⁶ unabhängig voneinander eine der in Anspruch 1 gegebenen Bedeutungen aufweisen, sowie Lewissäure-Addukte von Verbindungen der Formel (II.b).

9. Verbindungen der Formel (II.a) oder (II.b) sowie Lewissäure-Addukte von Verbindungen der Formel (II.b) nach Anspruch 8, worin R⁴ für -OR^{4a} steht, worin R^{4a} eine der zuvor gegebenen Bedeutungen besitzt.

10. Verwendung von Verbindungen der Formel (II.a) oder (II.b) sowie der Lewissäure-Addukte von Verbindungen der Formel (II.b), wie in einem der Ansprüche 8 bis 9 definiert, in einem Verfahren gemäß einem der Ansprüche 1 bis 6 zur Herstellung von Verbindungen der Formeln (I) oder (VI).

## Claims

1. A process for preparing compounds of the formula (I) 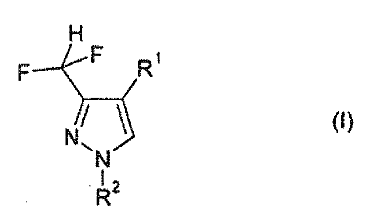 in which
R¹ is hydrogen, C₁-C₈-alkyl, phenyl, cyano or -C(=O)-OR^{1a}, where the phenyl group is unsubstituted or has 1 or 2 substituents selected independently from halogen, nitro, C₁-C₄-alkyl, C₁-C₄-haloalkyl and C₁-C₄-alkoxy, where
R^{1a} is C₁-C₈-alkyl, C₁-C₈-haloalkyl, C₃-C₈-cycloalkyl, C₁-C₄-alkoxy-C₁-C₄-alkyl, C₃-C₈-cycloalkoxy-C₁-C₄-alkyl, C₂-C₈-alkenyl, benzyl or phenyl, where the phenyl group in benzyl and phenyl is in each case unsubstituted or has 1, 2 or 3 substituents selected independently from halogen, CN, nitro, C₁-C₄-alkyl, C₁-C₄-haloalkyl, C₁-C₄-alkoxy and C₁-C₄-haloalkoxy;
R² is methyl;
comprising
A) the reaction of a compound of the formula (III) 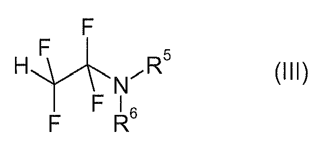 in which
R⁵ and R⁶ are each independently C₁-C₄-alkyl or, together with the nitrogen atom to which they are bonded, are an N-bonded 5- to 6-membered heterocycle which, as well as the nitrogen atom, may also have 1 or 2 further heteroatoms selected from N, O and S as ring atoms and is unsubstituted or has 1, 2, 3 or 4 substituents selected independently from halogen, CN, nitro, C₁-C₄-alkyl) C₁-C₄-haloalkyl); C₁-C₄-alkoxy and C₁-C₄-haloalkoxy;
with an acid and a compound of the formula (IV) 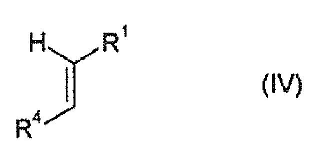 in which
R¹ is as defined above and
R⁴ is halogen, -OR^{4a}, -SR^{4a} or -O-SO₂-R^{4a}, in which
R^{4a} is hydrogen, C₁-C₈-alkyl, C₁-C₈-haloalkyl, C₂-C₈-alkenyl, C₃-C₈-cycloalkyl, benzyl or phenyl, where the phenyl group in benzyl and phenyl is in each case unsubstituted or has 1, 2 or 3 substituents selected independently from halogen, CN, nitro, C₁-C₄-alkyl, C₁-C₄-haloalkyl, C₁-C₄-alkoxy and C₁-C₄-haloalkoxy,
to obtain a reaction product (II), and
B) the reaction of the reaction product (II) with a hydrazine compound of the formula H₂N-NHR² in which R² has one of the definitions given above to obtain a compound of the formula (I).

2. The process according to claim 1, wherein the reaction product (II) prepared in step A is reacted without preceding isolation with the hydrazine compound of the formula H₂N-NHR² to prepare compounds of the formula (I).

3. The process according to either of the preceding claims, wherein the acid used for the reaction of the compound of the formula (III) is a Lewis acid.

4. The process according to claim 1, in which R⁴ is -OR^{4a} in which R^{4a} has one of the definitions given above.

5. A process for preparing compounds of the formula (VI) 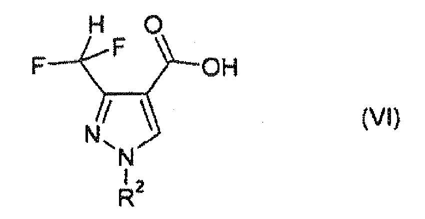 in which
R² is methyl;
comprising
i) the preparation of a compound of the formula (I.a) by a process according to the invention specified above for the preparation of compounds of the formula (I), 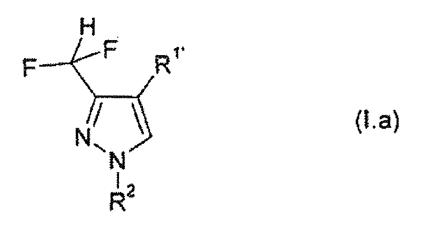 in which
R^{1'} is CN or -C(=O) -OR^{1aa}, where R1^{aa} is C₁-C₈- alkyl, C₁-C₈-haloalkyl, C₃-C₈-cycloalkyl, C₁-C₄-alkoxy-C₁-C₄-alkyl, C₃-C₈-cycloalkoxy- C₁-C₄-alkyl, C₂-C₈-alkenyl, benzyl or phenyl, and where the phenyl group in benzyl and phenyl is in each case unsubstituted or has 1, 2 or 3 substituents each independently selected from halogen, CN, nitro, C₁-C₄-alkyl, C₁-C₄-haloalkyl, C₁-C₄-alkoxy and C₁-C₄-haloalkoxy, and
R² has one of the definitions given above; and
ii) the hydrolysis of a compound of the formula (I.a) to obtain a compound of the formula (VI).

6. The process according to claim 5, in which the compound of the formla (I.a) prepared in step i) is used without preceding isolation in step ii) of the process according to the invention to prepare a compound of the formula (VI).

7. The use of compounds of the formula (III) as defined in claim 1 in a process according to any one of claims 1 to 6 for preparing compounds of the formula (I) or (VI) .

8. A compound of the formula (II.a) or (II.b), 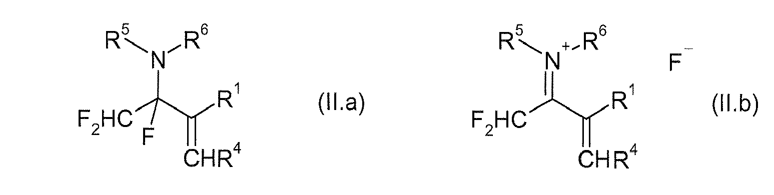 in which R¹, R⁴, R⁵ and R⁶ each independently have one of the definitions given in claim 1, or a Lewis acid adduct of a compound of the formula (II.b).

9. A compound of the formula (II.a) or (II.b) or a Lewis acid adduct of a compound of the formula (II.b) according to claim 8, in which R⁴ is -OR^{4a} in which R^{4a} has one of the definitions given above.

10. The use of compounds of the formula (II.a) or (II.b) or else of the Lewis acid adduct of a compound of the formula (II.b), as defined in any one of claims 8 to 9, in a process according to any one of claims 1 to 6 for preparing compounds of the formulae (I) or (VI) .

## Revendications

1. Procédé pour la préparation de composés de formule (I), 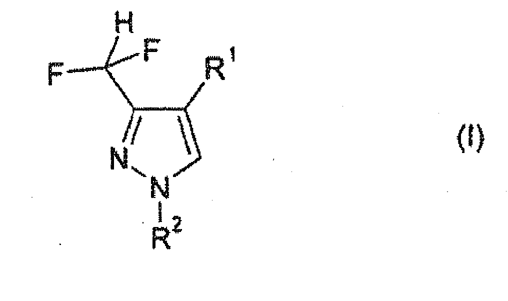 dans laquelle
R¹ représente un atome d'hydrogène, un groupe alkyle en C₁-C₈, phényle, cyano ou -C(=O)-OR^{1a}, le groupe phényle étant non substitué ou comportant 1 ou 2 substituants choisis, indépendamment l'un de l'autre, parmi des atomes d'halogène, des groupes nitro, alkyle en C₁-C₄, halogénoalkyle en C₁-C₄ et alcoxy en C₁-C₄,
R^{1a} représentant un groupe alkyle en C₁-C₈, halogénoalkyle en C₁-C₈, cycloalkyle en C₃-C₈, alcoxy(C₁-C₄)-alkyle(C₁-C₄), cycloalcoxy(C₃-C₈)-alkyle(C₁-C₄), alcényle en C₂-C₈, benzyle ou phényle, le groupe phényle dans les radicaux benzyle et phényle chaque fois étant non substitué ou comportant 1, 2 ou 3 substituants choisis chacun indépendamment parmi des atomes d'halogène, des groupes CN, nitro, alkyle en C₁-C₄, halogénoalkyle en C₁-C₄, alcoxy en C₁-C₄ et halogénoalcoxy en C₁-C₄ ; et
R² représente le groupe méthyle ;
comprenant
A) la mise en réaction d'un composé de formule (III) 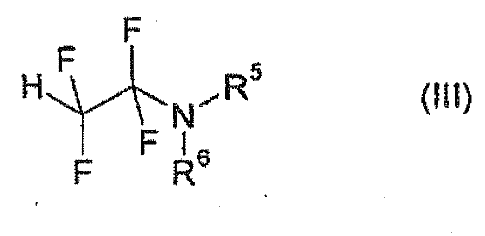 dans laquelle
R⁵ et R⁶ représentent, indépendamment l'un de l'autre, un groupe alkyle en C₁-C₄ ou représentent ensemble, avec l'atome d'azote auquel il sont fixés, un hétérocycle à 5 ou 6 chaînons, lié à N, qui peut comporter, en plus de l'atome d'azote, encore 1 ou 2 autres hétéroatomes choisis parmi N, O et S en tant qu'atomes formant le cycle, et est non substitué ou comporte 1, 2, 3 ou 4 substituants choisis, indépendamment les uns des autres, parmi des atomes d'halogène, des groupes CN, nitro, alkyle en C₁-C₄, halogénoalkyle en C₁-C₄, alcoxy en C₁-C₄ et halogénoalcoxy en C₂-C₄ ;
avec un acide et un composé de formule (IV) 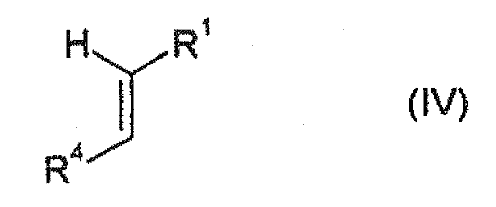 dans laquelle
R¹ a l'une des significations donnée précédemment et
R⁴ représente un atome d'halogène, -OR^{4a}, -SR^{4a} ou -O-SO₂-R^{4a}, où
R^{4a} représente un atome d'hydrogène, un groupe alkyle en C₁-C₈, halogénoalkyle en C₁-C₈, alcényle en C₂-C₈, cycloalkyle en C₃-C₈, benzyle ou phényle, le groupe phényle dans les radicaux benzyle et phényle chaque fois étant non substitué ou comportant 1, 2 ou 3 substituants choisis chacun indépendamment parmi des atomes d'halogène, des groupes CN, nitro, alkyle en C₁-C₄, halogénoalkyle en C₂-C₄, alcoxy en C₁-C₄ et halogénoalcoxy en C₁-C₄,
pour l'obtention d'un produit de réaction (II), et
B) la mise en réaction du produit de réaction (II) avec un composé de type hydrazine de formule H₂N-NHR² dans laquelle R² a l'une des significations données précédemment, avec obtention d'un composé de formule (I).

2. Procédé selon la revendication 1, dans lequel pour la préparation des composés de formule (I) on fait réagir le produit de réaction (II), préparé dans l'étape A, sans isolation préliminaire, avec le composé de type hydrazine de formule H₂N-NHR².

3. Procédé selon l'une quelconque des revendications précédentes, dans lequel l'acide utilisé dans la réaction du composé de formule (III) est un acide de Lewis.

4. Procédé selon la revendication 1, dans lequel R⁴ représente -OR^{4a}, où R^{4a} a l'une des significations données précédemment.

5. Procédé pour la préparation de composés de formule (VI), 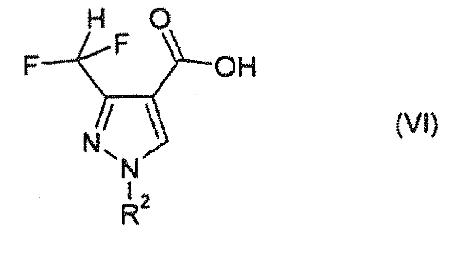 dans laquelle
R² représente le groupe méthyle ;
comprenant
i) la préparation d'un composé de formule (I.a) selon l'un des procédés conformes à l'invention, indiqués précédemment pour la préparation de composés de formule (I), 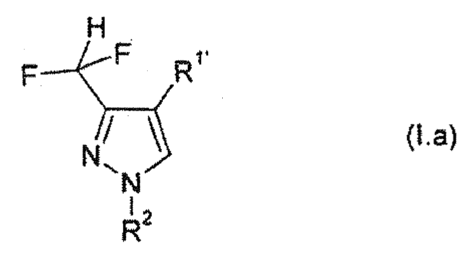 formule dans laquelle
R^{1'} représente CN ou -C(=O) -OR^{1aa}, R^{1aa} représentant un groupe alkyle en C₁-C₈, halogénoalkyle en C₁-C₈, cycloalkyle en C₃-C₈, alcoxy(C₁-C₄)- alkyle(C₁-C₄), cycloalcoxy(C₃-C₈)- alkyle(C₁-C₄), alcényle en C₂-C₈, benzyle ou phényle, le groupe phényle dans les radicaux benzyle et phényle chaque fois étant non substitué ou comportant 1, 2 ou 3 substituants choisis chacun indépendamment parmi des atomes d'halogène, des groupes CN, nitro, alkyle en C₁-C₄, halogénoalkyle en C₁-C₄, alcoxy en C₁-C₄ et halogénoalcoxy en C₁-C₄ ; et
R² a l'une des significations données précédemment ; et
ii) l'hydrolyse d'un composé de formule (I.a) avec obtention d'un composé de formule (VI).

6. Procédé selon la revendication 5, dans lequel le composé de formule (I.a) préparé dans l'étape i) est utilisé sans isolement préliminaire dans l'étape ii) du procédé selon l'invention pour la préparation d'un composé de formule (VI).

7. Utilisation de composés de formule (III), telle que définie dans la revendication 1, dans un procédé selon l'une quelconque des revendications 1 à 6 pour la préparation de composés de formules (I) ou (VI).

8. Composés de formules (II.a) ou (II.b), 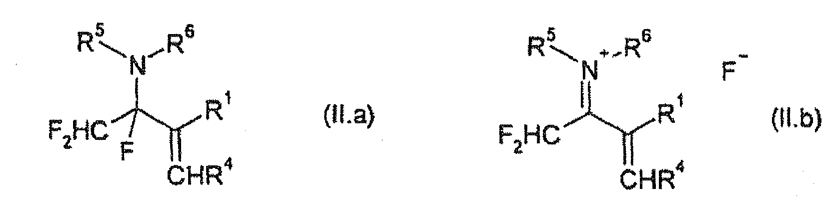 dans lesquelles R¹, R⁴ R⁵ et R⁶ ont, indépendamment les uns des autres, l'une des significations données dans la revendication 1, ainsi qu'adduits de Lewis de composés de formule (II.b).

9. Composés de formule (II.a) ou (II.b) ainsi qu'adduits de Lewis de composés de formule (II.b) selon la revendication 8, dans lesquels R⁴ représente -OR^{4a} où R^{4a} a l'une des significations données précédemment.

10. Utilisation de composés de formule (II.a) ou (II.b) ainsi que d'adduits de Lewis de composés de formule (II.b), telles que définies dans l'une quelconque des revendications 8 et 9, dans un procédé selon l'une quelconque des revendications 1 à 6 pour la préparation de composés de formules (I) ou (VI).
